# EUROPÄISCHE PATENTSCHRIFT

(11) **EP 3 823 770 B1**
(45) Veröffentlichungstag und Bekanntmachung des Hinweises auf die Patenterteilung: **22.06.2022**
(21) Anmeldenummer: 19769849.1
(22) Anmeldetag: 17.07.2019
(51) Int. Cl.: B08B 15/02, B01L 1/04, A61L 2/10, B65B 69/00, A61L 2/20, A61L 2/08, B08B 7/00

(54) **ANORDNUNG ZUM KONTAMINATIONSFREIEN EINSCHLEUSEN EINES STERILEN OBJEKTES AUS EINEM BEHÄLTNIS IN EIN CONTAINMENT UND VERFAHREN DAZU**
ARRANGEMENT FOR THE CONTAMINATION-FREE INTRODUCTION OF A STERILE OBJECT FROM A CONTAINER INTO A CONTAINMENT AND METHOD THEREFOR
DISPOSITIF POUR L'INFILTRATION SANS CONTAMINATION D'UN OBJET STÉRILE D'UN CONTENANT DANS UN CONFINEMENT ET PROCÉDÉ CORRESPONDANT

(30) Priorität: 18.07.2018 CH 8882018
(43) Veröffentlichungstag der Anmeldung: 26.05.2021
(73) Patentinhaber: Pharma Integration S.R.L., 53100 Siena (IT)
(72) Erfinder: LANDES, Robert, 79713 Bad Säckingen (DE); MÜLLER, Mathieu, 68510 Sierentz (FR); SPRECHER, Kathrin, 4055 Basel (CH); ZELLER, Mike, 4246 Wahlen (CH)
(74) Vertreter: Dall'Olio, Christian
(86) Internationale Anmeldenummer: PCT/IB2019/000620
(87) Internationale Veröffentlichungsnummer: WO 2020/016645

(56) Entgegenhaltungen:
- EP-A1- 3 263 297
- WO-A1-2018/019782
- H.J. BAESSLER, F. LEHMAN: "Containment Technology", 978-3-662-51052-0, 1. Januar 2013 (2013-01-01), Seiten 81-104, XP009512843, Berlin-Heidelberg ISBN: 978-3-642-39291-7 in der Anmeldung erwähnt

## Beschreibung

### Anwendungsgebiet der Erfindung

Die Erfindung betrifft eine Anordnung sowie ein Verfahren zum kontaminationsfreien Einschleusen eines sterilisierten Objektes aus einem mit einer Abdeckung verschlossenen Behältnis in eine von einer Wandung umgebenen Arbeitskammer eines Containments. Typisch in der Labortechnik, der Pharmaindustrie oder der Biotechnik haben solche Behältnisse die Gestalt wannenförmiger Tubs und die Objekte sind Nester mit einem Raster von muldenförmigen Aufnahmekonturen zur Lagerung von Containern, z.B. Phiolen oder Spritzen.

### Stand der Technik

Nicht nur das gesicherte Ausschleusen von Objekten aus einem Containment erfordert spezielle Vorrichtungen, wie z.B. in der WO 2010/145 042 A1 offenbart, sondern auch das Einschleusen von Objekten in ein Containment. Beim Einschleusen muss zunächst vermieden werden, das Containment nach aussen zu öffnen, damit sowohl der reinraumtechnisch definierte Zustand im Containment erhalten bleibt, aber auch keine gefährlichen Partikel aus dem Containment nach aussen gelangen. Ferner müssen beim Einschleusen von sterilisierten Objekten diese soweit geschützt sein, dass während des Einschleusens die Objekte selber keiner Verunreinigung aus der Umgebung ausgesetzt sind.

In der Monographie von H.-J. Bässler und F. Lehman: Containment Technology.

Springer-Verlag, Berlin, Heidelberg, 2013, S. 88ff., welche eine Anordnung gemäß dem Oberbegriff des Anspruchs 1 offenbart, wird ein die vorgenannten Bedingungen erfüllendes Transfersystem (Rapid Transfer Port - RTP) beschrieben. Hierbei ist in der Wandung des Containments, zumeist in der Art eines kreisrunden Fensters, ein Port, vorgesehen, der von einem abgedichteten Innenflansch umrandet wird. In der Ausgangssituation ist dieser Innenflansch mit einem in das Innere des Containments zu öffnenden und gegen den Innenflansch dichten Türelement verschlossen. Das in das Containment zu transferierende Objekt - z.B. ein steriles pharmazeutisches Produkt - ist in einer zunächst vom Containment gelösten, aussen dichten Kapsel eingeschlossen, an der die Mündung von einem abgedichteten Aussenflansch umrandet ist, auf dem ein dicht schliessender Deckel aufsitzt. In einem ersten Transferschritt wird die Kapsel an dem Verbund aus Innenflansch und Türelement angedockt, so dass Innenflansch mit Aussenflansch sowie Türelement mit Deckel miteinander dicht verriegelt sind und das Türelement mit dem Deckel deren beide unsterilen Aussenflächen umschliesst. Im nächsten Transferschritt wird der Verbund aus Türelement und Deckel vom Verbund beider Flansche in das Innere des Containments bewegt, so dass die Mündung der Kapsel jetzt offen ist und das sterile Objekt vom Container in das Innere des Containments gebracht werden kann.

Im Prinzip äquivalent verläuft das bisherige Handling, wenn das in das Containment einzuschleusende Produkt, ein verschlossenes Behältnis mit einem darin vorhandenen sterilen Objekt ist. Eine derartige Konstellation ergibt sich, wenn das Behältnis die Gestalt eines in der Medizintechnik sogenannten Tubs hat, das z.B. mit einer Abdeckung aus Vliesgewebe, wie Tyvek^{®}, versiegelt ist und sich im Behältnis das sterile Objekt befindet, welches die sogenannte Gestalt eines Nestes hat, in dem eine Vielzahl von Phiolen oder medizinischen Spritzen deponiert ist. Bei Nutzung des oben beschriebenen Transfersystems für das zuvor definierte Behältnis (versiegeltes Tub mit innerem Nest, bestückt z.B. mit Phiolen) stehen zwei alternative Wege zur Verfügung. Beim ersten Weg müsste das Behältnis mit Inhalt auch äusserlich dekontaminiert sein bevor es in die Kapsel kommt oder das Behältnis wird in der Kapsel dekontaminiert, um nach dem Überführen in das Containment dort das sterile Objekt auszupacken. Der zweite Weg wäre das Objekt unter sterilen Bedingungen aus dem Behältnis zu entpacken, so in die Kapsel einzubringen und schliesslich in das Containment zu transferieren. Eine andere Alternative ist, das Objekt ungeschützt auszupacken und dann in der Kapsel zu dekontaminieren und schliesslich in das Containment zu transferieren. Eine weitere Methode ist, das Behältnis unter kontrollierten Bedingungen aus den Umhüllungen zu entnehmen und anschliessend, um eine potentielle zwischenzeitliche Kontamination zu beseitigen, wird das Behältnis mit der dieses verschliessenden Abdeckung allseitig in einer E-Beam-Anlage bestrahlt. Dann erfolgt die Überführung in das Containment, die Abdeckung wird entfernt, das Objekt entnommen und die darin magazinierten Container werden vereinzelt.

Die obigen Lösungen sind im Handling umständlich und erfordern für das Auspacken des Objektes erhöhten apparativen Aufwand im Containment bzw. ausserhalb.

### Aufgabe der Erfindung

Angesichts der bisher unvollkommenen Lösungen liegt der Erfindung die Aufgabe zugrunde, eine Anordnung zu schaffen, die zum kontaminationsfreien Einschleusen eines sterilen Objektes aus einem Behältnis in ein Containment bestimmt ist, und ein Verfahren mit Benutzung der geschaffenen Anordnung vorzuschlagen. Zielstellung hierbei ist es, das Handling möglichst einfach bei Gewährleistung der Reinheitserfordernisse und optimiertem Vorrichtungsaufwand zu gestalten. Speziell gilt es, möglichst nur jene Materialien und Gerätschaften in das Containment einzubringen, welche zum unmittelbaren Bearbeitungsprozess benötigt werden, und zugleich den erforderlichen Umfang an nötiger Dekontamination zu minimieren.

### Übersicht über die Erfindung

Die der Erfindung zugrunde liegende Aufgabe wird durch eine Anordnung gemäß Anspruch 1 gelöst. Diese Anordnung enthält:
a) eine Toreinheit mit:
   aa) einem in der Wandung angeordneten Zugangsflansch, der einen Durchgang von aussen in die Arbeitskammer ausbildet; und
   ab) einer den Durchgang dicht verschliessenden Tür, die sich zur Offenstellung in die Arbeitskammer bewegen lässt; und
b) eine Behältnisaufnahme mit:
   ba) einem Auffang zur Halterung eines in die Behältnisaufnahme eingebrachten Behältnisses welches mit einer Abdeckung verschlossen ist;
   bb) einer Öffnung zum Einbringen des Behältnisses in den Auffang; und
   bc) einem Flansch zum Zusammenwirken mit dem Zugangsflansch.

Eine Dekontaminationseinheit ist dazu bestimmt, bei geschlossener Tür, am Zugangsflansch angedockter Behältnisaufnahme und in der Behältnisaufnahme gelagertem Behältnis, eine der Tür zugewandte Aussenfläche der Abdeckung zu dekontaminieren.

Nachfolgend werden spezielle Ausführungsformen der Erfindung definiert: Die Tür ist in Schliessstellung von Seiten der Arbeitskammer abgedichtet an den Zugangsflansch angefügt, und der Zugangsflansch umrandet einen Zwischenraum, der im Durchgang liegt. Die Abdeckung des in der Behältnisaufnahme eingestellten Behältnisses ist bei an den Zugangsflansch herangeschwenkter Behältnisaufnahme zwischen dem Flansch und dem Zugangsflansch abgedichtet.

Die Dekontaminationseinheit, mit Wirkungsrichtung in den Durchgang hinein, ist direkt an der Tür oder seitlich einer Toreinheit installiert, welche die Tür und den Zugangsflansch aufweist. Die Dekontaminationseinheit ist eine Strahlungsquelle, z.B. UVC, UV, E-Beam oder Puls-Licht, oder eine Begasungseinrichtung, z.B zur Verneblung einer H₂O₂-Lösung. Die Dekontaminationseinheit ist ferner zur Dekontamination der nach aussen gewandten Oberfläche der Tür, des Zwischenraums und der vom Zugangsflansch dem Zwischenraum zugewandten Fläche nutzbar.

Ein in der Arbeitskammer mit einem Werkzeugkopf versehenes Transfergerät ist dazu bestimmt, bei geöffneter Tür, am Zugangsflansch angedockter Behältnisaufnahme und in der Behältnisaufnahme gelagertem Behältnis:
a) den dekontaminierten Teil der Abdeckung vom Behältnis zu lösen und in die Arbeitskammer zu bringen;
b) eine im Behältnis optional vorhandene, über dem Objekt liegende Einlage in die Arbeitskammer zu bringen; und
c) aus dem in der Behältnisaufnahme liegenden Behältnis das darin ruhende Objekt in die Arbeitskammer zu bringen.

Bei Verwendung der Anordnung in der Labortechnik, der Pharmaindustrie oder der Biotechnik:
a) hat das Behältnis die Gestalt eines wannenförmigen Tubs; und
b) hat das Objekt die Gestalt eines Nestes mit einem Raster von muldenförmigen Aufnahmekonturen zur Lagerung von Containern, z.B. Phiolen oder Spritzen.

Der Anordnung ist eine Eingangsstation mit einem durch einen Filter fliessenden Luftstrom zugeordnet, welche zum Freisetzen der Behältnisse aus mit, z.B. tütenartigen Umhüllungen versehenen Packungen und zur Bereitstellung der Behältnisse unter kontrollierten reinraumtechnischen Bedingungen, vor dem Einstellen in die Behältnisaufnahme, bestimmt ist.

Die das Behältnis verschliessende Abdeckung, z.B. aus Vliesgewebe, wie Tyvek^{®}, bewahrt das im Innenraum des Behältnisses liegende Objekt mit den Containern in sterilem Zustand. Die das Behältnis verschiessende Abdeckung ist vorzugsweise an einem Behältnisrand des Behältnisses versiegelt.

Das Verfahren **,** gemäß Anspruch 8, zum kontaminationsfreien Einschleusen eines sterilen Objektes aus einem Behältnis in ein Containment unter Verwendung der vorgenannten Anordnung umfasst die Abfolge nachstehender Prozessphasen:
a) Entnehmen des mit einer Abdeckung verschlossenen und ein Objekt enthaltenden Behältnisses aus Umhüllungen unter kontrollierten reinraumtechnischen Bedingungen an einer Eingangsstation;
b) Bereitstellung des jeweils einzelnen mit einer Abdeckung verschlossenen Behältnisses mit darin gelagertem sterilem Objekt zum kontaminationsfreien Einschleusen in eine Arbeitskammer eines Containments; hierbei sind:
   - der Durchgang zur Arbeitskammer nach aussen offen, aber in das Containment von der Tür verschlossen, die Dekontaminationseinheit wirkungslos, der Auffang der Behältnisaufnahme offen und leer, die Behältnisaufnahme von der Toreinheit gelöst;
c) Einstellen des bereitgestellten einzelnen Behältnisses in den Auffang; hierbei sind:
   - weiterhin der Durchgang nach aussen offen, aber in das Containment von der Tür verschlossen, die Dekontaminationseinheit wirkungslos und die Behältnisaufnahme von der Toreinheit gelöst, nun aber der Auffang mit einem Behältnis gefüllt;
c) Heranfahren der Behältnisaufnahme an die Toreinheit; hierbei sind:
   - der Zugangsflansch der Toreinheit mit dem Flansch gegeneinander abgedichtet;
   - weiterhin der Durchgang in das Containment von der Tür verschlossen, die Dekontaminationseinheit wirkungslos und der Auffang mit einem Behältnis gefüllt; und
   - der Durchgang auch nach aussen verschlossen, nämlich von der an der Toreinheit abgedichtet angedockten Behältnisaufnahme;
d) die Dekontaminationseinheit aktivschalten bzw. in Wirkungszustand bringen; hierbei sind:
   - weiterhin der Zugangsflansch der Toreinheit mit dem Flansch gegeneinander abgedichtet, der Durchgang in das Containment von der Tür verschlossen, der Auffang mit einem Behältnis gefüllt und der Durchgang auch nach aussen verschlossen, nämlich von der an der Toreinheit abgedichtet angedockten Behältnisaufnahme; und nun
   - die zur Tür exponierte Aussenfläche der Abdeckung sowie der Zwischenraum und alle diesem zugewandten Oberflächen steril gemacht;
e) die Dekontaminationseinheit abschalten bzw. ausser Wirkungszustand bringen und Öffnen der Tür; hierbei sind:
   - weiterhin der Zugangsflansch der Toreinheit mit dem Flansch gegeneinander abgedichtet, der Auffang mit einem Behältnis gefüllt, der Durchgang nach aussen verschlossen, nämlich von der an der Toreinheit dicht angedockten Behältnisaufnahme, und die Aussenfläche der Abdeckung, der Zwischenraum sowie alle diesem zugewandten Oberflächen steril; und nun
   - der Durchgang in das Containment offen;
f) Aufschneiden der Abdeckung des Behältnisses; hierbei sind:
   - weiterhin der Zugangsflansch der Toreinheit mit dem Flansch gegeneinander abgedichtet, der Auffang mit einem Behältnis gefüllt, der Durchgang nach aussen verschlossen, nämlich von der an der Toreinheit dicht angedockten Behältnisaufnahme, die Aussenfläche der Abdeckung, der Zwischenraum sowie alle diesem zugewandten Oberflächen steril und der Durchgang in das Containment offen; und nun
   - der Zugang zum im Behältnis liegenden Objekt offen;
g) Entnehmen des Objekts aus dem Behältnis und Überführen des Objekts mit den darin gespeicherten Containern in die Arbeitskammer des Containments zur weiteren Bearbeitung; hierbei sind:
   - weiterhin der Zugangsflansch der Toreinheit mit dem Flansch gegeneinander abgedichtet, der Durchgang nach aussen verschlossen, nämlich von der an der Toreinheit dicht angedockten Behältnisaufnahme und der Durchgang in das Containment offen; und nun
   - der Auffang nur mehr mit dem leeren Behältnis gefüllt; und
h) Rückführung der Anordnung in die Ausgangsphase, d.h. Bewegen der Tür in die Schliessstellung, welche damit wieder gegen den Zugangsflansch abgedichtet ist, Abschwenken der Behältnisaufnahme vom Zugangsflansch und Entfernen des leeren Behältnisses aus dem Auffang.

Nachfolgend werden spezielle Prozessphasen erwähnt: Nach dem Heranfahren der Behältnisaufnahme mit dem im Auffang eingestellten Behältnis an die Toreinheit und vor dem Aktivschalten der Dekontaminationseinheit bzw. Versetzen in ihren Wirkungszustand, wird das Objekt einem Integritätstest unterzogen, um festzustellen, ob der Inhalt des Behältnisses das Reinheitserfordernis erfüllt. Falls der Integritätstest ein Negativergebnis zeigt, wird die Behältnisaufnahme von der Toreinheit abgeschwenkt, um das quasi schadhafte Behältnis aus dem Auffang zu entfernen.

Das Aufschneiden der Abdeckung des Behältnisses, die Entnahme des Objekts aus dem Behältnis und das Überführen des Objekts mit den darin gespeicherten Containern in die Arbeitskammer des Containments erfolgt mittels des in der Arbeitskammer positionierten Transfergeräts.

Ein freigeschnittener Teil der Abdeckung wird mittels des in der Arbeitskammer positionierten Transfergeräts in die Arbeitskammer gebracht.

Das einzelne Behältnis, welches mit der Abdeckung verschlossen ist, mit seinem sterilen Inhalt, nämlich dem im Behältnis gelagerten Objekt und den darin aufgenommenen Containern, ist mit zumindest einer Umhüllung versehen und bildet so im Anlieferungszustand eine Packung.

Die Bereitstellung des einzelnen Behältnisses vor dem Einstellen in die Behältnisaufnahme erfolgt in einer zur Anordnung gehörenden Eingangsstation als Freisetzen des Behältnisses aus der jeweiligen Packung unter kontrollierten reinraumtechnischen Bedingungen.

### Kurzbeschreibung der beigefügten Zeichnungen

Es zeigen:

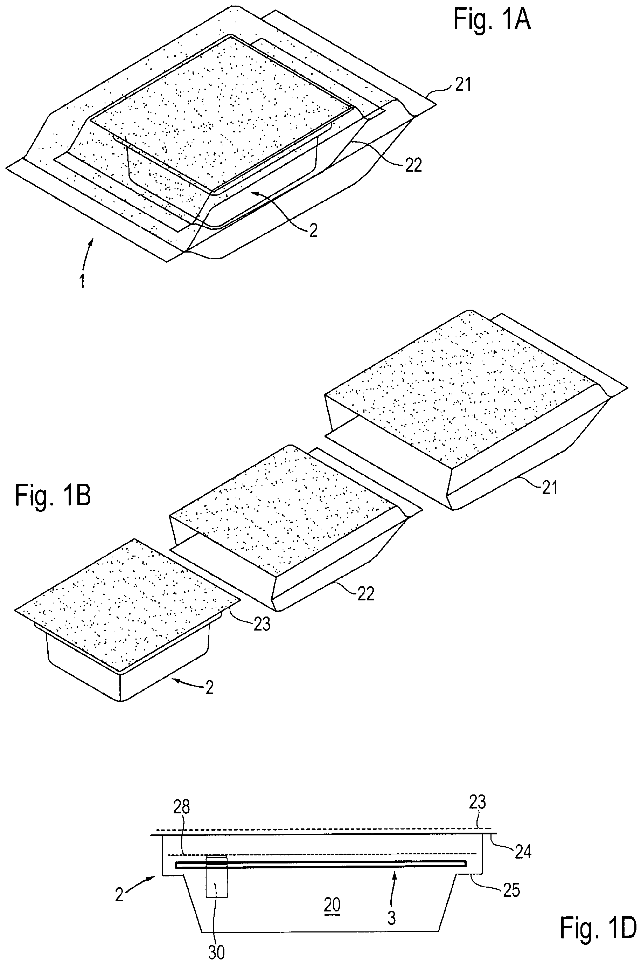

- Figur 1A -: eine Packung, bestehend aus gefülltem Behältnis mit Umhüllungen, in transparenter Perspektivansicht;
- Figur 1B -: das Behältnis und die separaten Umhüllungen aus Figur 1A, in Perspektivansicht;

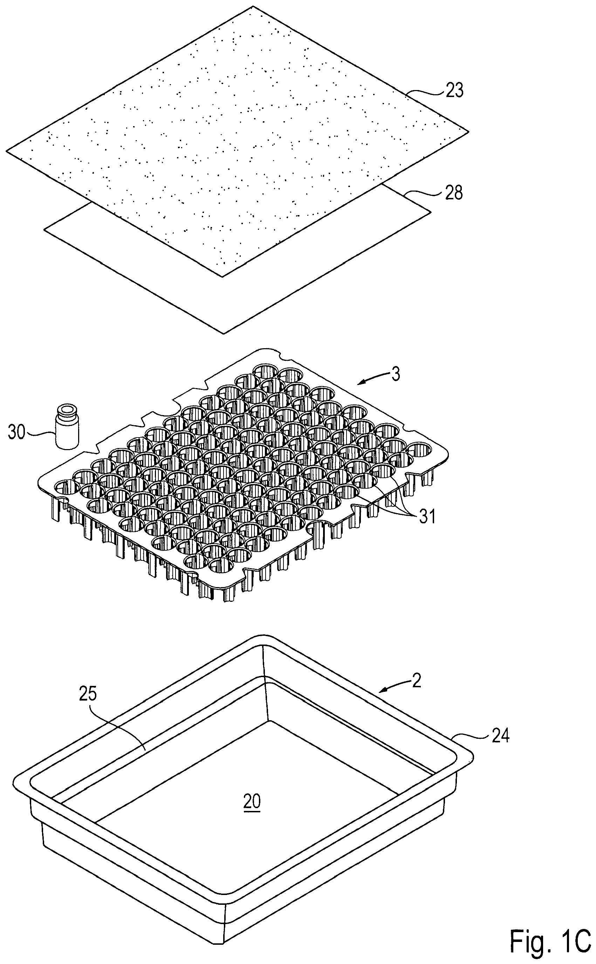

- Figur 1C -: das Behältnis aus Figur 1A, mit einem Objekt in Gestalt eines Nestes zur Aufnahme einer Vielzahl von Containern, einem einzelnen Container, einer Einlage und einer Abdeckung, in prinzipieller Explosivansicht;
- Figur 1D -: die Teile gemäss Figur 1C, ineinander gestellt, in transparenter Frontansicht;

Figuren 2A bis 10B: die erfindungsgemässe Anordnung zum kontaminationsfreien Einschleusen eines Objektes in ein Containment mit Toreinheit, Eingangsstation und Behältnisaufnahme, dazu die Abfolge der Prozessphasen, in Prinzipdarstellungen;

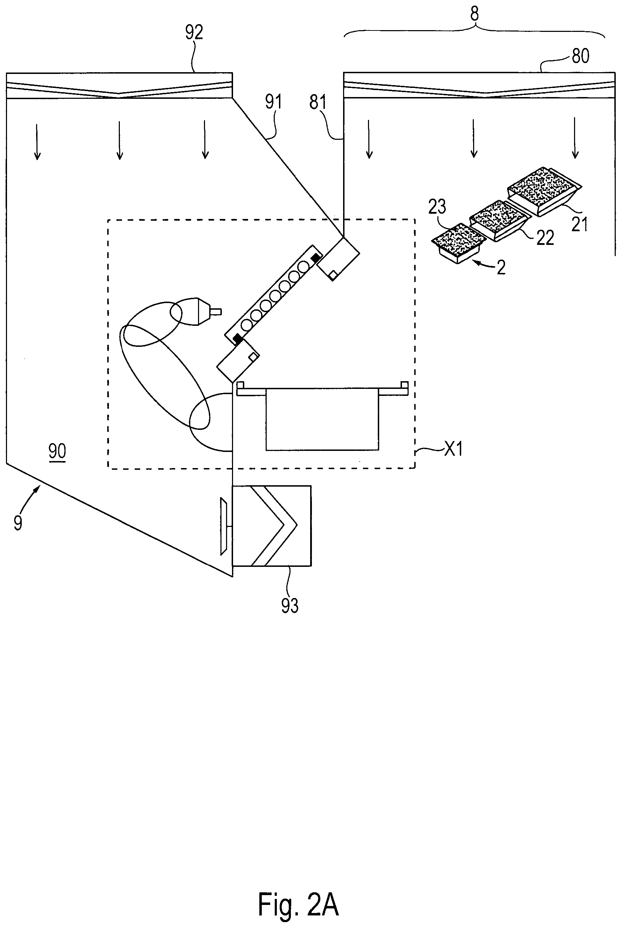

- Figur 2A -: Prozessphase 1: Entnehmen des mit einer Abdeckung verschlossenen und ein Objekt enthaltenden Behältnisses aus den Umhüllungen unter kontrollierten reinraumtechnischen Bedingungen an einer Eingangsstation;

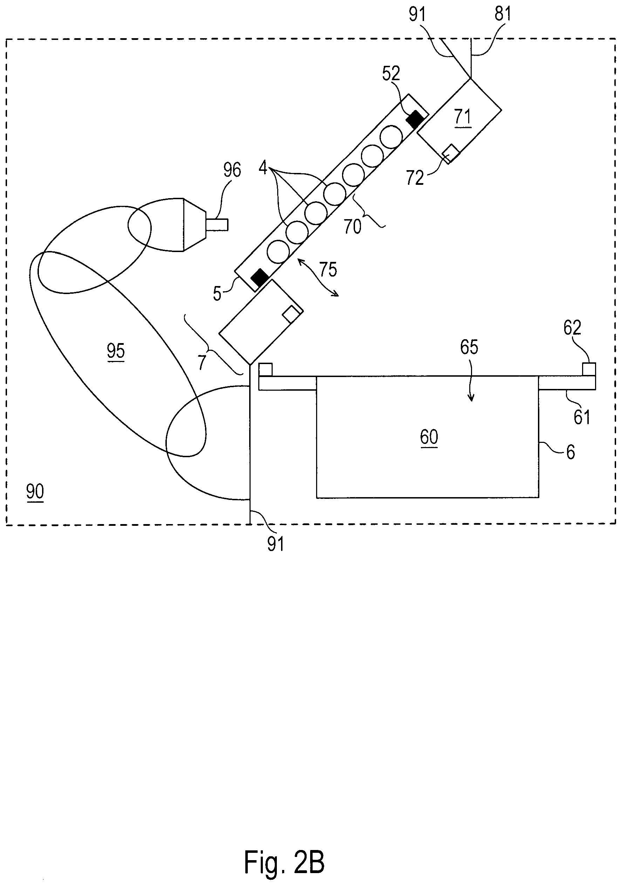

- Figur 2B -: das vergrösserte Detail X1 aus Figur 2A;

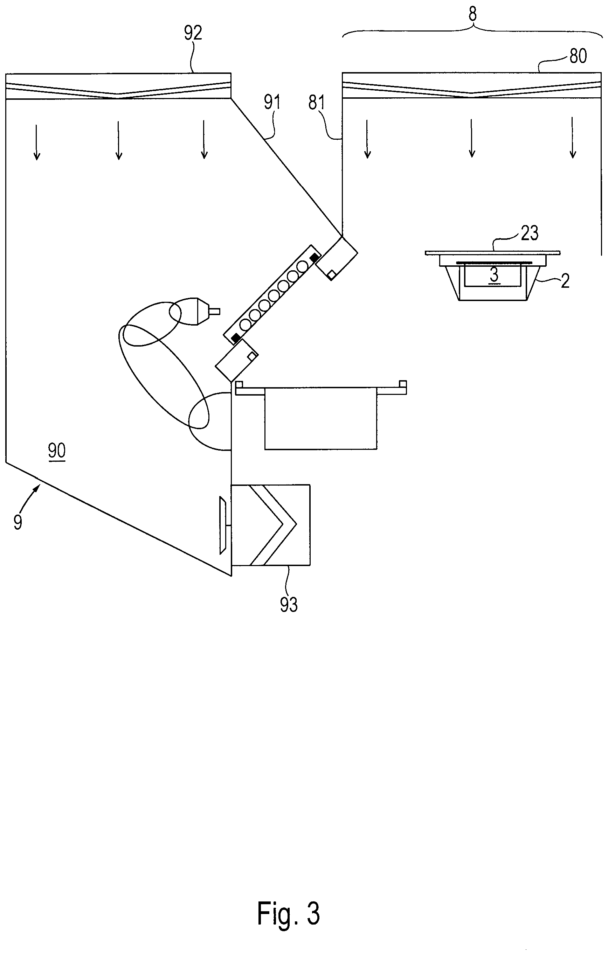

- Figur 3 -: Prozessphase 2: Bereitstellung des Behältnisses zum Einschleusen, Durchgang in Containment von Tür verschlossen, Behältnisaufnahme leer und von Toreinheit gelöst;

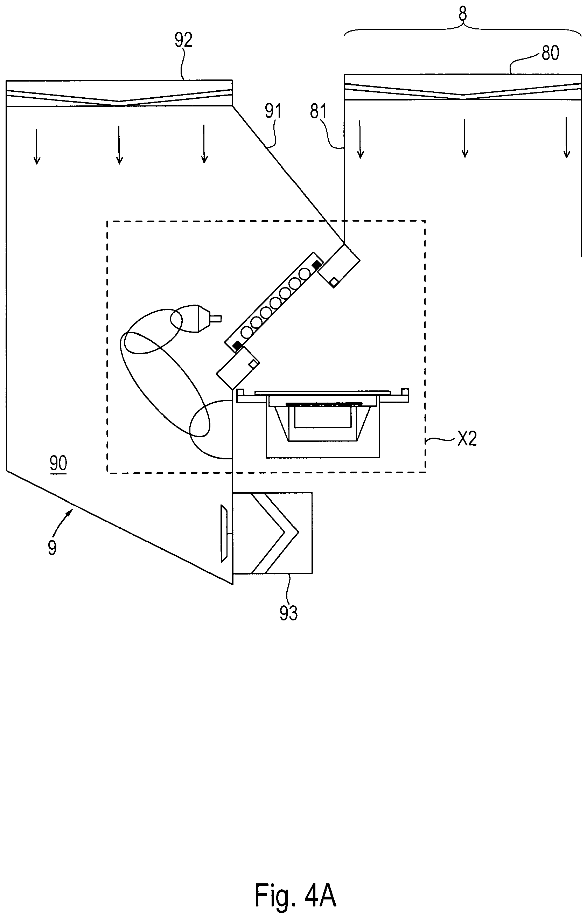

- Figur 4A -: Prozessphase 3: Einstellen des Behältnisses in Behältnisaufnahme;

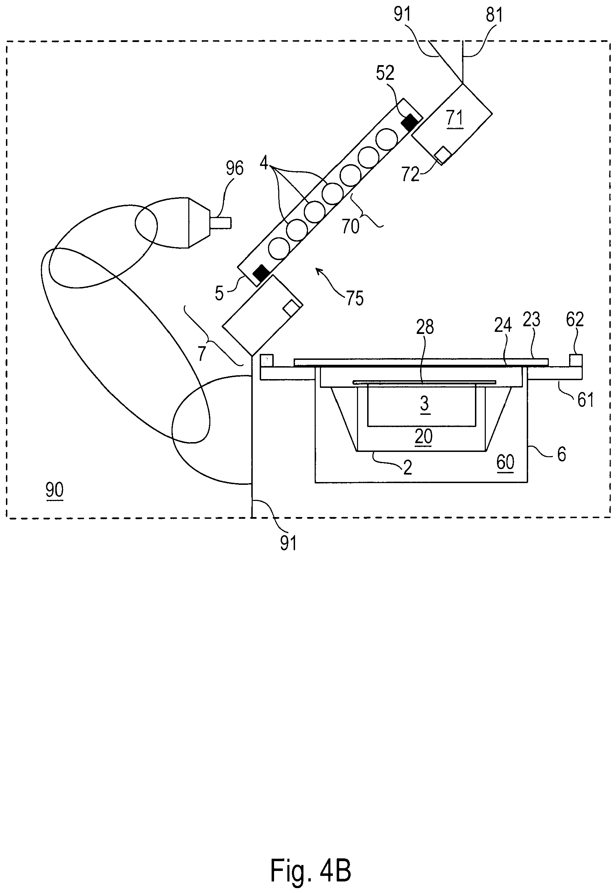

- Figur 4B -: das vergrösserte Detail X2 aus Figur 4A;

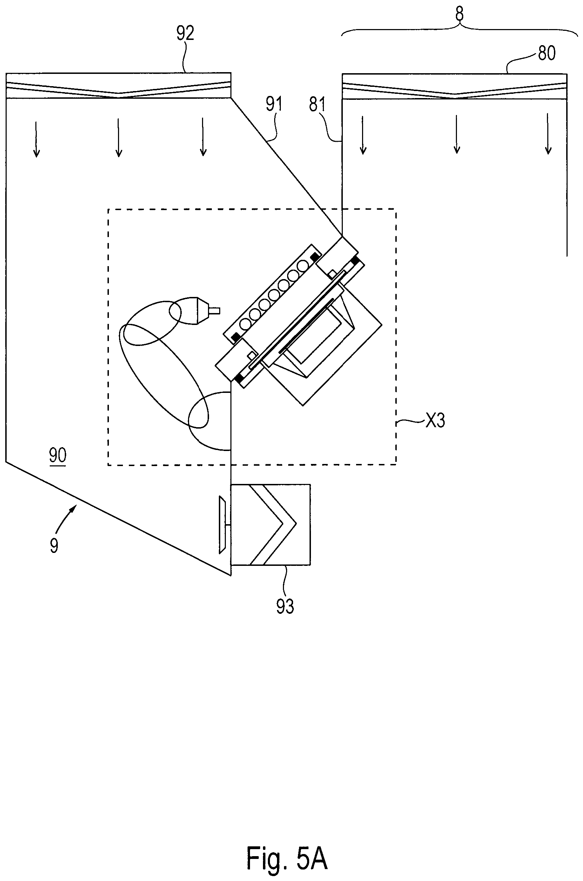

- Figur 5A -: Prozessphase 4: Heranfahren der Behältnisaufnahme an Toreinheit und Verschluss zwischen Zugangsflansch und Flansch;

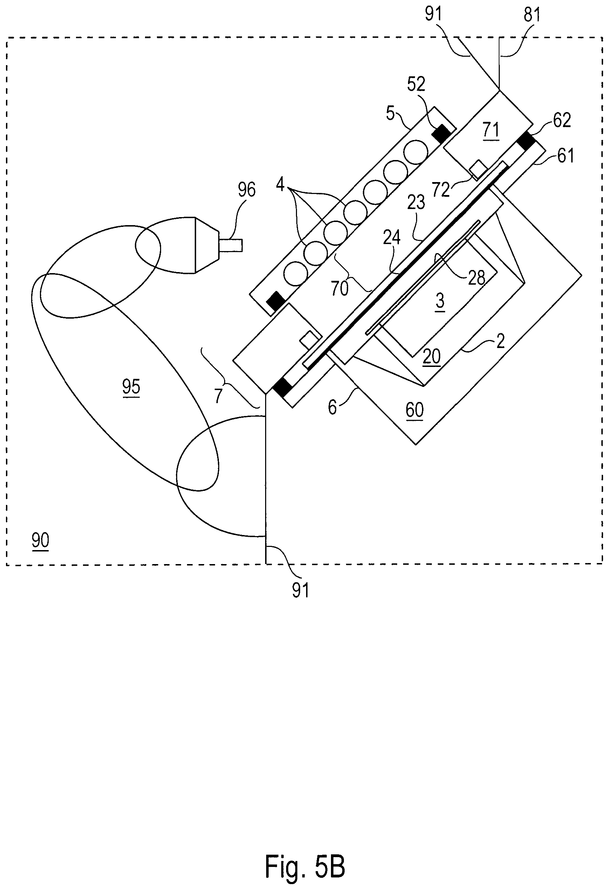

- Figur 5B -: das vergrösserte Detail X3 aus Figur 5A;

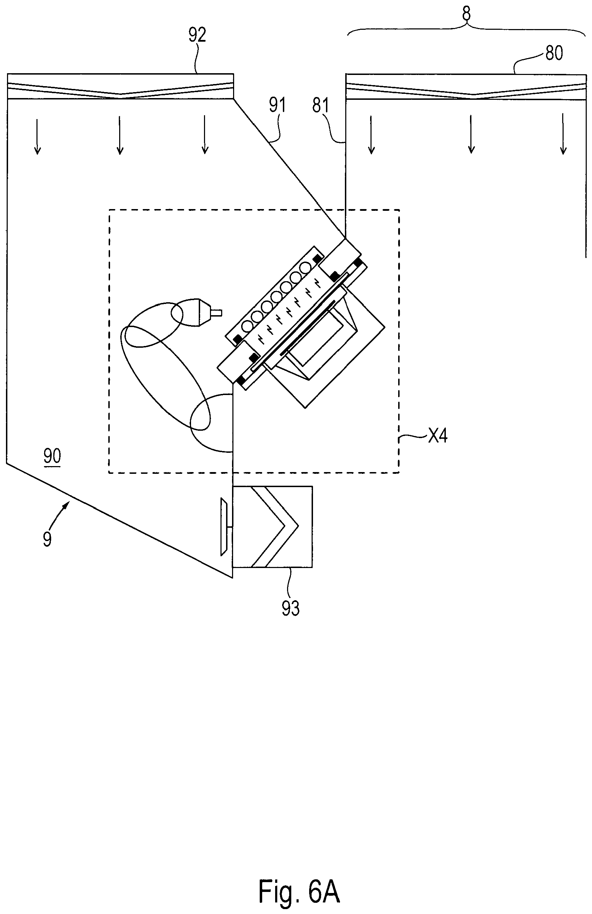

- Figur 6A -: Prozessphase 5: nach eventuellem Integritätstest des Objektes, aktivschalten der Dekontaminationseinheit;

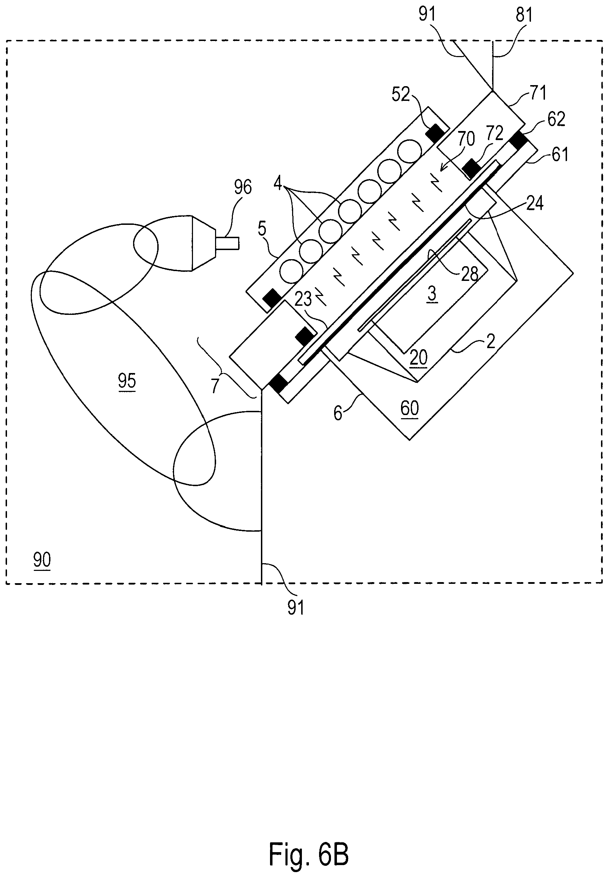

- Figur 6B -: das vergrösserte Detail X4 aus Figur 6A;

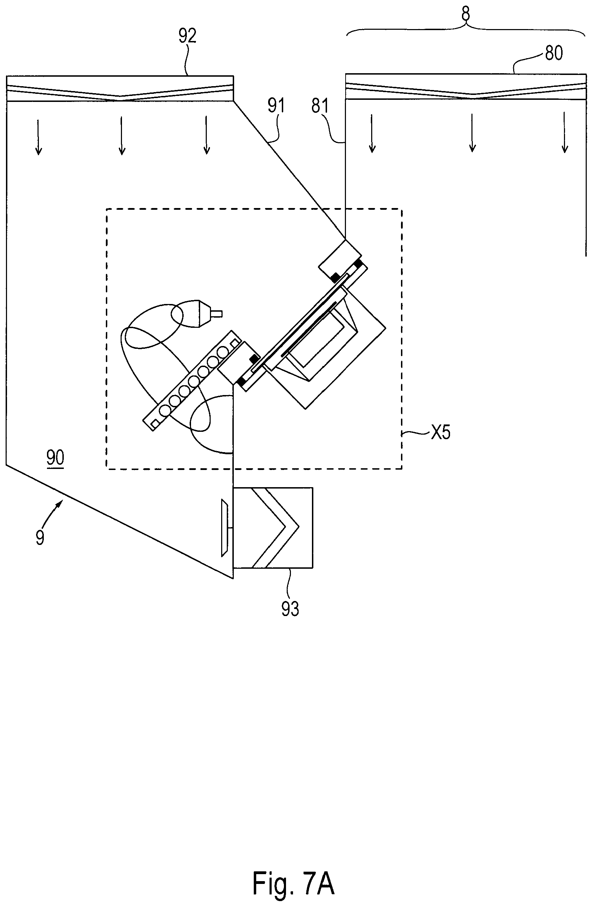

- Figur 7A -: Prozessphase 6: Passivschalten der Dekontaminationseinheit, Öffnen der Tür, von Seiten des Containments, offener Zugang zum Zwischenraum bis an die Abdeckung;

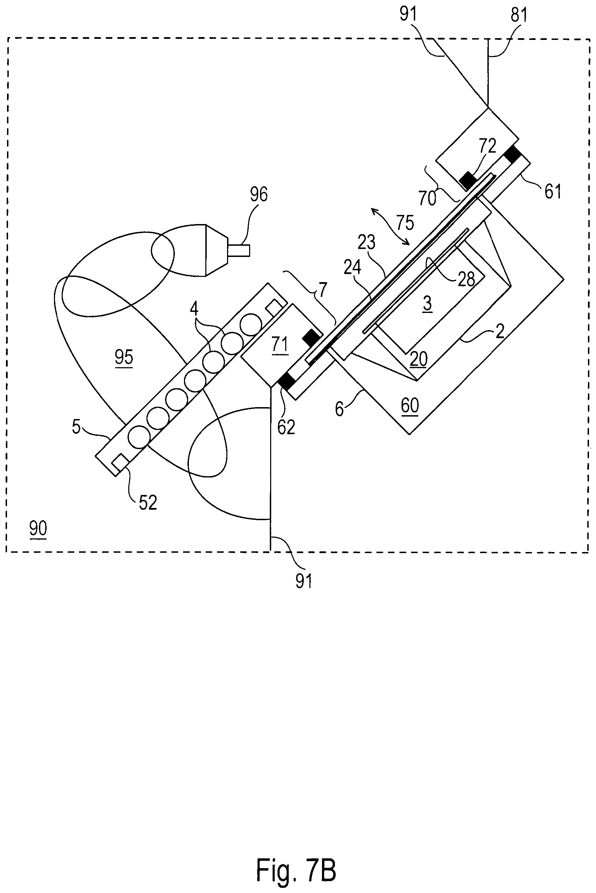

- Figur 7B -: das vergrösserte Detail X5 aus Figur 7A;

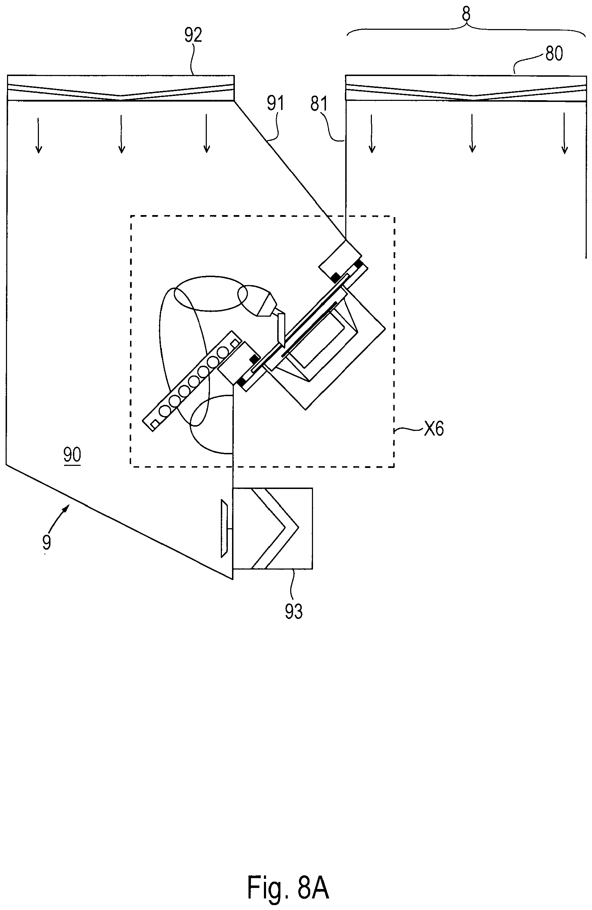

- Figur 8A -: Prozessphase 7: Transfergerät mit Trennwerkzeug am Werkzeugkopf bestückt fährt in Zwischenraum ein, Freischneiden der Abdeckung des Behältnisses;

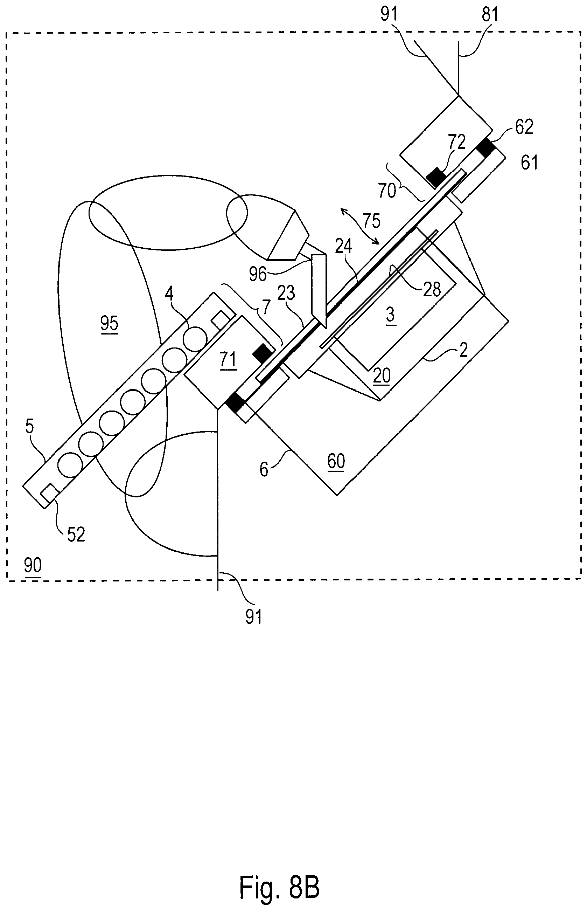

- Figur 8B -: das vergrösserte Detail X6 aus Figur 8A;

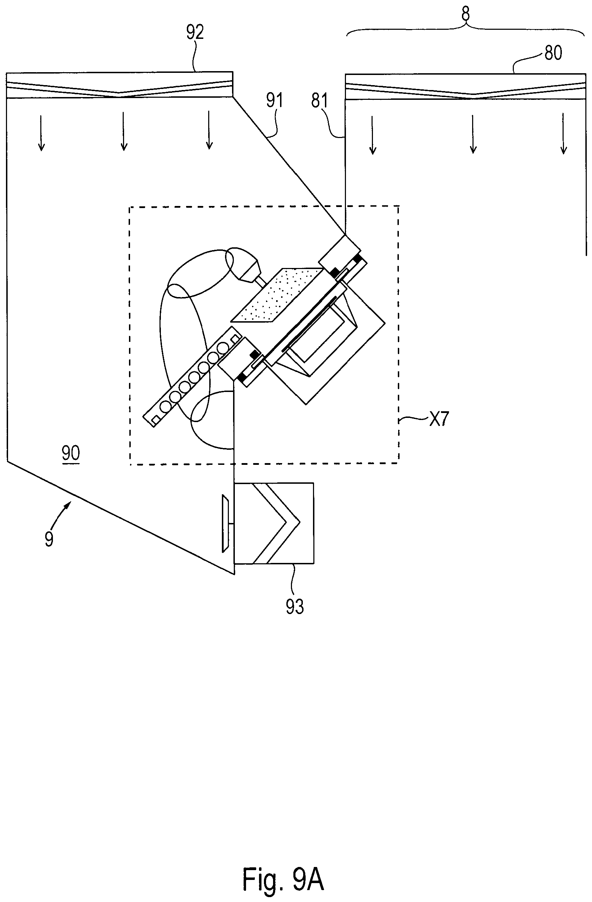

- Figur 9A -: Prozessphase 8: Transfergerät entfernt Freischnitt der Abdeckung und die eventuell vorhandene Einlage;

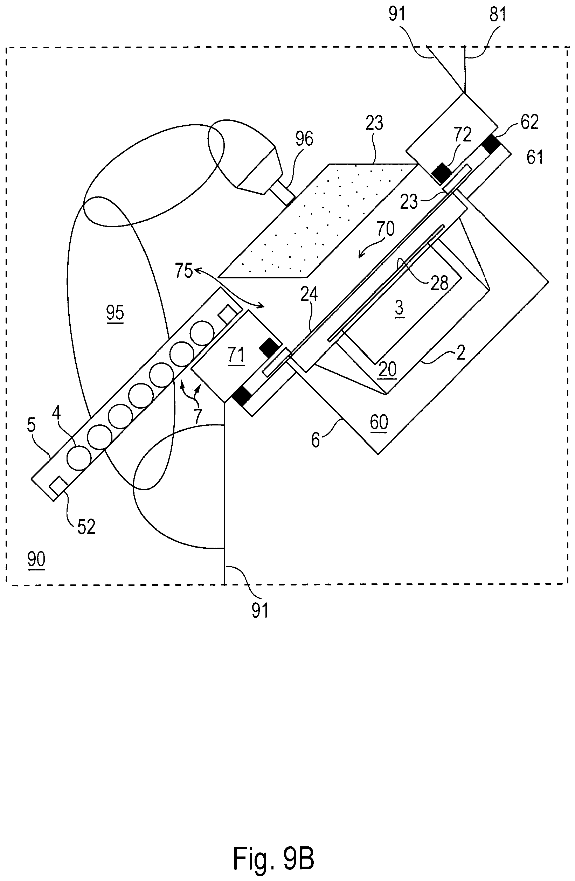

- Figur 9B -: das vergrösserte Detail X7 aus Figur 9A;

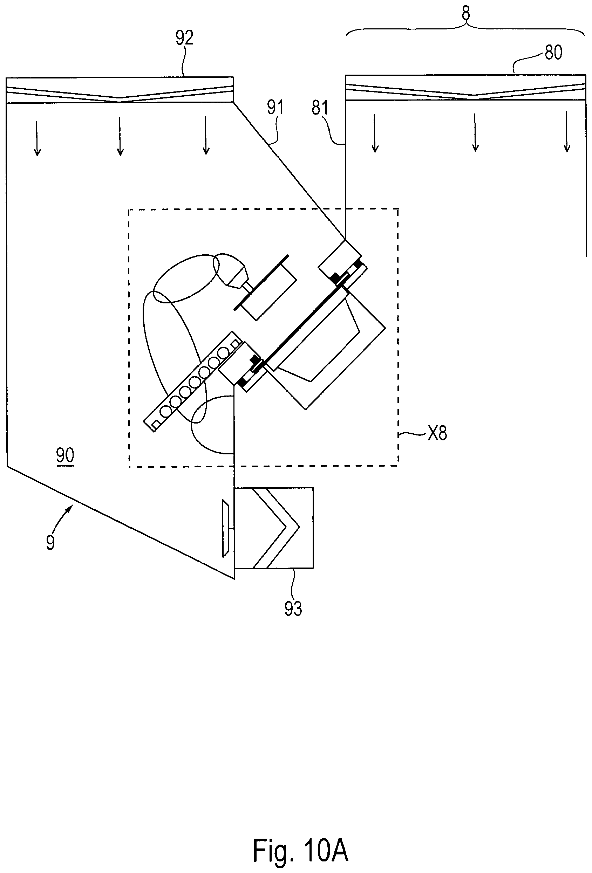

- Figur 10A -: Prozessphase 9: Transfergerät hebt Objekt aus Behältnis und bringt Objekt in Arbeitskammer des Containments zur weiteren Bearbeitung; und

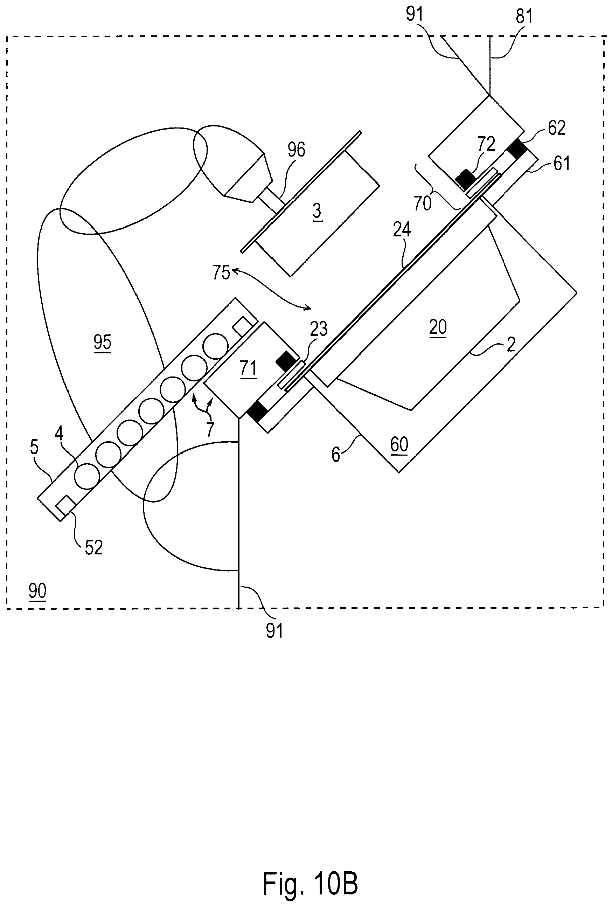

- Figur 10B -: das vergrösserte Detail X8 aus Figur 10A.

### Ausführungsbeispiel

Mit Bezug auf die beiliegenden Zeichnungen erfolgt nachstehend die detaillierte Beschreibung der erfindungsgemässen Anordnung zum kontaminationsfreien Einschleusen eines sterilen Objektes aus einem Behältnis in ein Containment, beginnend mit einer Erläuterung zum strukturellen Aufbau von Behältnis und darin enthaltenem Objekt. Hierbei wird das Einschleusen anhand der illustrierten sukzessiven Abfolge der Prozessphasen geschildert, so dass die prinzipielle Zusammensetzung der Anordnung und zugleich die mit Benutzung der Anordnung durchgeführten Verfahrensschritte zum Einschleusen erklärt sind.

Für die gesamte weitere Beschreibung gilt folgende Festlegung: Sind in einer Figur zum Zweck zeichnerischer Eindeutigkeit Bezugsziffern enthalten, aber im unmittelbar zugehörigen Beschreibungstext nicht erläutert, so wird auf deren Erwähnung in vorangehenden oder nachfolgenden Figurenbeschreibungen Bezug genommen.

### Figuren 1A bis 1D

Im Anlieferungszustand ergibt das in einer ersten äusseren Umhüllung **21** und eventuell auch in einer inneren zweiten Umhüllung **22** steckende Behältnis **2** die Packung **1.** Beide Umhüllungen **21,22** sind jeweils tütenartigen ausgebildet, verschlossen und vorzugsweise aus einem Vliesgewebe oder mit einem Vliesfenster versehen. Das z.B. wannenförmig beschaffene und typischerweise aus Kunststoff bestehende Behältnis **2** hat zuoberst eine Abdeckung **23,** zumeist ebenfalls aus Vliesgewebe, wie Tyvek^{®}, die auf dem Behältnisrand **24** versiegelt ist. Alternativ hat die Abdeckung **23** eine anteilige Fläche aus Vliesgewebe, um zu ermöglichen, dass gasförmiges Dekontaminationsmittel an das Objekt **3** und die Container **30** gelangen kann.

Im Innenraum **20** des Behältnisses **2** befindet sich eine umlaufende Stützschulter **25,** auf welcher das im Innenraum **20** untergebrachte Objekt **3** quasi hängt, das mit einer Vielzahl von Containern **30** bestückt ist. In der Pharmaindustrie ist für das Behältnis **2** die Bezeichnung "Tub" und für das Objekt **3** die Bezeichnung "Nest" gebräuchlich, während die Container **30** z.B. die Gestalt von Phiolen oder Spritzen haben. Das Objekt **3** besitzt systematisch positionierte und zu den Containern **30** komplementär gestaltete Aufnahmekonturen **31** zur geordneten Anordnung der Container **30.** Oben auf dem Objekt **3** könnte lose eine blattförmige Einlage **28** liegen, wiederum aus Vliesgewebe. Das gesamte Innere der Packung **1,** also das Behältnis **2,** sein Innenraum **20,** die Innenflächen, die Unterseite der Abdeckung **23,** die Einlage **28** sowie das Objekt **3** und die Container **30** wurden durch Behandlung steril gemacht.

### Figuren 2A bis 10B

Anhand dieser Aufreihung von Figurenpaaren, welche den prinzipiellen Aufbau der Vorrichtung und die sukzessive Abfolge der Prozessphasen 1 bis 9 beim Einschleusen illustrieren, erfolgt nun die zugehörige detailliert erklärende Beschreibung. Hier werden bei der Abhandlung der jeweils nächsten Prozessphase nur die Änderungen zur vorangehenden charakterisiert.

### Figur 2A und 2B - Aufbau der Vorrichtung und Prozessphase 1

Im für die hiesige Erläuterung nur fragmentarisch dargestellten Containment **9** befindet sich die von der Wandung **91** umgebene Arbeitskammer **90,** über der ein Eingangsfilter **92** zwecks Zuführung eines gereinigten Luftstroms in die Arbeitskammer **90** angeordnet ist. Die Arbeitskammer **90** ist mit einem Transfergerät **95** ausgestattet, das zuvorderst einen Werkzeugkopf **96** hat. Im unteren Bereich der Arbeitskammer **90** ist ein Abluftfilter **93** eingebaut.

In der Wandung **91** ist eine Toreinheit **7** installiert, die einen eine erste Dichtung **72** aufweisenden Zugangsflansch **71** hat, welcher einen Zwischenraum **70** umrandet. Sofern nicht einerseits von der geschlossenen Tür **5** und/oder andererseits von der heranbewegten Behältnisaufnahme **6** abgesperrt, bildet dieser Zwischenraum **70** einen Durchgang **75** von aussen zur Arbeitskammer **90.** Die mit einer zweiten Dichtung **52** versehene Tür **5** ist innerhalb der Arbeitskammer **90** vorgesehen und lässt sich von einer an den Zugangsflansch **71** ansetzenden Schliessstellung in eine in die Arbeitskammer **90** hinein geschwenkte Offenstellung bewegen. Die Tür **5** ist zugleich Träger einer Dekontaminationseinheit **4,** vorzugsweise mittels UVC-Strahlung. Die Behältnisaufnahme **6** ist nahe der Toreinheit **7,** aussen am Containment **9,** vorzugsweise an den Zugangsflansch **71** heranschwenkbar, positioniert und hat den durch die Öffnung **65** zugänglichen Auffang **60** und einen Flansch **61** mit der daran vorhanden dritten Dichtung **62.**

In vereinfachter Konstruktion lassen sich die erste Dichtung **72** am Zugangsflansch **71** und die zweite Dichtung **52** an der Tür **5** zu einer gemeinsamen, am Zugangsflansch **71** angeordneten Dichtung **72/52** kombinieren. Eine so kombinierte Dichtung **72/52** umfasst den Zugangsflansch **71** an seinen dem Zwischenraum **70** zugewandten Innenflächen und wirkt somit einerseits zwischen der geschlossenen Tür **5** und dem Zugangsflansch **71** sowie andererseits zwischen der herangeschwenkten Behältnisaufnahme **6** und dem Zugangsflansch **71.**

An das Containment **9** angrenzend befindet sich die Eingangsstation **8** mit dem oben installierten Filter **80** und den Seitenwandungen **81** zur Leitung eines vom Filter **80** erzeugten abwärts fliessenden Luftstromes. Die Eingangsstation **8** ermöglicht das Entnehmen des mit der Abdeckung **23** verschlossenen und ein Objekt **3** enthaltenden Behältnisses **2** aus den Umhüllungen **21,22** unter kontrollierten reinraumtechnischen Bedingungen und die Bereitstellung des Behältnisses **2** zum Einschleusen.

In Prozessphase 1 besteht folgende Situation:
- In der Eingangsstation **8,** unter dem durch den Filter **80** fliessenden Luftstrom werden von der Packung **1** die Umhüllungen **21,22** unter kontrollierten reinraumtechnischen Bedingungen entfernt und das entnommene Behältnis **2** bereitgestellt. Die versiegelte Abdeckung **23** bewahrt das im Innenraum **20** liegende Objekt **3** mit den Containern **30** in sterilem Zustand. Die äussere Oberfläche der Abdeckung **23** jedoch ist jedenfalls nach der Entnahme aus den Umhüllungen **21,22** und durch den Kontakt mit der Atmosphäre nicht mehr garantiert steril.
- Die Tür **5** steht mittels der zweiten Dichtung **52** abgedichtet am Zugangsflansch **71** in Schliessstellung und die Dekontaminationseinheit **4** sowie das Transfergerät **95** sind inaktiv.
- Die Behältnisaufnahme **6** mit leerem Auffang **60** ist vom Zugangsflansch **71** abgeschwenkt.
- Der Durchgang **75** in die Arbeitskammer **90** ist von der verschlossenen Tür **5** abgesperrt und der Zwischenraum **70** nach aussen hin offen.

### Figur 3 - Prozessphase 2

Nun besteht folgende Situation:
- Das komplette, nun entpackte Behältnis **2** mit der dieses verschliessenden Abdeckung **23** und dem im Behältnis **2** gelagerten Objekt **3** befindet sich bereit zum Einschleusen in der Eingangsstation **8.**

### Figuren 4A und 4B - Prozessphase 3

Nun besteht folgende Situation:
- Das komplette Behältnis **2** ist von der Eingangsstation **8** durch die Öffnung **65** in die weiterhin vom Zugangsflansch **71** abgeschwenkte Behältnisaufnahme **6** eingestellt.
- Vorzugsweise überragt der Behältnisrand **24** die Öffnung **65,** so dass sich der Behältnisrand **24** auf dem Flansch **61** abstützt und das Behältnis **2** im Auffang **60** hängt.

### Figuren 5A und 5B - Prozessphase 4

Nun besteht folgende Situation:
- Die Behältnisaufnahme **6** mit dem darin aufgenommenen Behältnis **2** ist an den Zugangsflansch **71** herangeschwenkt. Hierbei bewirken die dritte Dichtung **62** die sichere Abdichtung zwischen Flansch **61** und Zugangsflansch **71** und die erste Dichtung **72** die sichere Abdichtung zwischen Flansch **61** und Behältnisrand **24** bzw. der darauf, vorzugsweise versiegelten Abdeckung **23.**
- Der Durchgang **75** in die Arbeitskammer **90** ist weiterhin von der verschlossenen Tür **5** abgesperrt, jetzt aber auch nach aussen hin von der herangeschwenkten Behältnisaufnahme **6,** so dass der Zwischenraum **70** nun allseits hermetisch isoliert ist.
- Der Inhalt des Behältnisses **2** kann einem Integritätstest unterzogen werden, um vor Fortsetzung des Prozesses festzustellen, dass der Inhalt das Reinheitserfordernis erfüllt.
- Die erste Dichtung **72** wird aktiviert.

### Figuren 6A und 6B - Prozessphase 5

Nun besteht folgende Situation:
- Die Dekontaminationseinheit **4** kommt zur aktiven Wirkung, um dem Zwischenraum **70,** alle diesem zugewandten Oberflächen, d.h. die innere Ringfläche des Zwischenflansches **71,** die Aussenfläche der Abdeckung **23** und die eventuelle freie Fläche an der ersten Dichtung **72** steril zu machen.

### Figuren 7A und 7B - Prozessphase 6

Nun besteht folgende Situation:
- Die Dekontaminationseinheit **4** wird abgeschaltet bzw. wird im verbleibenden eingeschalteten Zustand zurückgezogen.
- Die Tür **5** wird durch Bewegen in das Innere der Arbeitskammer **90** geöffnet, so dass aus dem Inneren der Arbeitskammer **90** heraus der Durchgang **75** in den Zwischenraum 70 bis an die Abdeckung **23** heran zugänglich ist.

### Figuren 8A und 8B - Prozessphase 7

Nun besteht folgende Situation:
- Das Trennwerkzeug am Werkzeugkopf **96** des Transfergeräts **95** kommt zum Einsatz und schwenkt zum Zwischenraum **70,** um die Abdeckung **23** freizuschneiden, damit das Objekt **3** aus dem Behältnis **2** entnehmbar wird.

### Figuren 9A und 9B - Prozessphase 8

Nun besteht folgende Situation:
- Das Greifwerkzeug am Werkzeugkopf **96** des Transfergeräts **95** kommt zum Einsatz, um die freigeschnittene Abdeckung **23** und eventuell auch die auf dem Objekt **3** oben aufliegende Einlage **28** in das Innere der Arbeitskammer **90** zu bringen.

### Figuren 10A und 10B - Prozessphase 9

Nun besteht folgende Situation:
- Mit dem Greifwerkzeug am Werkzeugkopf **96** des Transfergeräts **95** wird das Objekt **3** erfasst, aus dem Behältnis **2** gehoben und in die Arbeitskammer **90** bewegt.
- Das Objekt **3** mit den darin gespeicherten Containern **30** wird dem weiteren Bearbeitungsprozess im Containment **9** zugeführt.
- Das leere Objekt **3** kann in das Behältnis **2** zurückplatziert werden.
- Die Anordnung wird in die Prozessphase 1 bzw. 2 gebracht, d.h. die Tür **5** wird in die Schliessstellung bewegt und ist damit wieder gegen den Zugangsflansch **71** abgedichtet. Die Behältnisaufnahme **6** wird vom Zugangsflansch **71** abgeschwenkt, um das nun leere bzw. mit dem leeren Objekt 3 beladene Behältnis **2** aus der Behältnisaufnahme **6** zu entfernen.

## Patentansprüche

1. Anordnung mit einer von einer Wandung **(91)** umgebenen Arbeitskammer **(90)** eines Containments **(9)** und bestimmt zum kontaminationsfreien Einschleusen eines sterilen Objekts **(3)** in die Arbeitskammer **(90),** umfassend:
a) eine Toreinheit **(7)** mit:
aa) einem in der Wandung **(91)** angeordneten Zugangsflansch **(71),** der einen Durchgang **(75)** von aussen in die Arbeitskammer **(90)** ausbildet; und
ab) einer den Durchgang **(75)** dicht verschliessenden Tür **(5),** die sich zur Offenstellung in die Arbeitskammer **(90)** bewegen lässt;
b) eine Behältnisaufnahme **(6)** mit:
ba) einem durch eine Öffnung **(65)** zugänglichen Auffang (60) zur Halterung eines in die Behälteraufnahme (6) eingebrachten Behältnisses (2) welches mit einer Abdeckung (23) verschlossen ist; und
bb) einem Flansch **(61)** zum Zusammenwirken mit dem Zugangsflansch **(71); dadurch gekennzeichnet, dass**
c) die Anordnung eine Dekontaminationseinheit (4) umfasst, wobei
d) die Dekontaminationseinheit **(4)** derart ausgeführt ist, bei geschlossener Tür **(5),** am Zugangsflansch **(71)** angedockter Behältnisaufnahme **(6)** und in der Behältnisaufnahme **(6)** gelagertem Behältnis **(2),** eine der Tür **(5)** zugewandte Aussenfläche der Abdeckung (**23**) des Behältnisses (2) zu dekontaminieren.

2. Anordnung nach Anspruch 1, **dadurch gekennzeichnet, dass**
a) die Tür **(5)** in Schliessstellung von Seiten der Arbeitskammer **(90)** abgedichtet an den Zugangsflansch **(71)** angefügt ist und der Zugangsflansch **(71)** einen Zwischenraum **(70)** umrandet, der im Durchgang **(75)** liegt; und
b) die Abdeckung (**23**), welche vorzugsweise semipermeabel ist, des in der Behältnisaufnahme **(6)** eingestellten Behältnisses **(2)** bei an den Zugangsflansch **(71)** herangeschwenkter Behältnisaufnahme **(6)** zwischen dem Flansch **(61)** und dem Zugangsflansch **(71)** abgedichtet ist.

3. Anordnung nach zumindest einem der Ansprüche 1 oder 2, **dadurch gekennzeichnet, dass** die Dekontaminationseinheit **(4),** mit Wirkungsrichtung in den Durchgang **(75)** hinein, direkt an der Tür **(5)** oder seitlich der Toreinheit **(7)** installiert ist, welche die Tür **(5)** und den Zugangsflansch **(71)** aufweist.

4. Anordnung nach zumindest einem der Ansprüche 1 bis 3, **dadurch gekennzeichnet, dass** die Dekontaminationseinheit **(4)** eine Strahlungsquelle, z.B. UVC, UV, E-Beam oder Puls-Licht, oder eine Begasungseinrichtung, z.B zur Verneblung einer H₂O₂-Lösung ist.

5. Anordnung nach zumindest einem der Ansprüche 2 bis 4, **dadurch gekennzeichnet, dass** die Dekontaminationseinheit **(4)** ferner zur Dekontamination der nach aussen gewandten Oberfläche der Tür **(5),** des Zwischenraums **(70)** und der vom Zugangsflansch **(71)** dem Zwischenraum **(70)** zugewandten Fläche nutzbar ist.

6. Anordnung nach zumindest einem der Ansprüche 1 bis 5, **dadurch gekennzeichnet, dass** ein in der Arbeitskammer **(90)** mit einem Werkzeugkopf **(96)** versehenes Transfergerät **(95)** dazu bestimmt ist, bei geöffneter Tür **(5),** am Zugangsflansch **(71)** angedockter Behältnisaufnahme **(6)** und in der Behältnisaufnahme **(6)** gelagertem Behältnis **(2):**
a) den dekontaminierten Teil der Abdeckung **(23)** vom Behältnis **(2)** zu lösen und in die Arbeitskammer **(90)** zu bringen;
b) eine im Behältnis **(2)** optional vorhandene, über dem Objekt **(3)** liegende Einlage **(28)** in die Arbeitskammer **(90)** zu bringen; und
c) aus dem in der Behältnisaufnahme **(6)** liegenden Behältnis **(2)** das darin ruhende Objekt **(3)** in die Arbeitskammer **(90)** zu bringen.

7. Anordnung nach zumindest einem der Ansprüche 1 bis 6, **dadurch gekennzeichnet, dass** der Anordnung eine Eingangsstation **(8)** mit einem durch einen Filter **(80)** fliessenden Luftstrom zugeordnet ist, bestimmt zum Freisetzen der Behältnisse **(2)** und zur Bereitstellung der Behältnisse **(2)** unter kontrollierten reinraumtechnischen Bedingungen, vor dem Einstellen in die Behältnisaufnahme **(6).**

8. Verfahren zum kontaminationsfreien Einschleusen eines sterilen Objektes **(3)** aus einem Behältnis **(2)** in ein Containment **(9)** unter Verwendung der Anordnung gemäss zumindest einem der vorherigen Ansprüche, wobei das Verfahren die Abfolge nachstehender Prozessphasen umfasst:
a) Entnehmen des mit einer Abdeckung **(23)** verschlossenen und ein Objekt **(3)** enthaltenden Behältnisses **(2)** aus Umhüllungen **(21,22)** unter kontrollierten reinraumtechnischen Bedingungen an einer Eingangsstation **(8);**
b) Bereitstellung des jeweils einzelnen mit der Abdeckung **(23)** verschlossenen Behältnisses **(2)** mit darin gelagertem sterilem Objekt **(3)** zum kontaminationsfreien Einschleusen in eine Arbeitskammer **(90)** eines Containments **(9);** hierbei sind:
- der Durchgang **(75)** zur Arbeitskammer **(90)** nach aussen offen, aber in das Containment **(9)** von der Tür **(5)** verschlossen, die Dekontaminationseinheit **(4)** wirkungslos, der Auffang **(60)** der Behältnisaufnahme **(6)** offen und leer, die Behältnisaufnahme **(6)** von der Toreinheit **(7)** gelöst;
c) Einstellen des bereitgestellten einzelnen Behältnisses **(2)** in den Auffang **(60);** hierbei sind:
- weiterhin der Durchgang **(75)** nach aussen offen, aber in das Containment **(9)** von der Tür **(5)** verschlossen, die Dekontaminationseinheit **(4)** wirkungslos und die Behältnisaufnahme **(6)** von der Toreinheit **(7)** gelöst, nun aber der Auffang **(60)** mit einem Behältnis **(2)** gefüllt;
d) Heranfahren der Behältnisaufnahme **(6)** an die Toreinheit **(7);** hierbei sind:
- der Zugangsflansch **(71)** der Toreinheit **(7)** mit dem Flansch **(61)** gegeneinander abgedichtet;
- weiterhin der Durchgang **(75)** in das Containment **(9)** von der Tür **(5)** verschlossen, die Dekontaminationseinheit **(4)** wirkungslos und der Auffang **(60)** mit einem Behältnis **(2)** gefüllt; und
- der Durchgang **(75)** auch nach aussen verschlossen, nämlich von der an der Toreinheit **(7)** abgedichtet angedockten Behältnisaufnahme **(6);**
e) die Dekontaminationseinheit **(4)** aktivschalten bzw. in Wirkungszustand bringen; hierbei sind:
- weiterhin der Zugangsflansch **(71)** der Toreinheit (7) mit dem Flansch **(61)** gegeneinander abgedichtet, der Durchgang **(75)** in das Containment **(9)** von der Tür **(5)** verschlossen, der Auffang **(60)** mit einem Behältnis **(2)** gefüllt und der Durchgang **(75)** auch nach aussen verschlossen, nämlich von der an der Toreinheit **(7)** abgedichtet angedockten Behältnisaufnahme **(6);** und nun
- die zur Tür **(5)** exponierte Aussenfläche der Abdeckung **(23)** sowie der Zwischenraum **(70)** und alle diesem zugewandten Oberflächen steril gemacht;
f) die Dekontaminationseinheit **(4)** abschalten bzw. ausser Wirkungszustand bringen und Öffnen der Tür **(5);** hierbei sind:
- weiterhin der Zugangsflansch **(71)** der Toreinheit **(7)** mit dem Flansch **(61)** gegeneinander abgedichtet, der Auffang **(60)** mit einem Behältnis **(2)** gefüllt, der Durchgang **(75)** nach aussen verschlossen, nämlich von der an der Toreinheit **(7)** dicht angedockten Behältnisaufnahme **(6),** und die Aussenfläche der Abdeckung **(23),** der Zwischenraum **(70)** sowie alle diesem zugewandten Oberflächen steril; und nun
- der Durchgang **(75)** in das Containment **(9)** offen;
g) Aufschneiden der Abdeckung **(23)** des Behältnisses **(2);** hierbei sind:
- weiterhin der Zugangsflansch **(71)** der Toreinheit **(7)** mit dem Flansch **(61)** gegeneinander abgedichtet, der Auffang **(60)** mit einem Behältnis **(2)** gefüllt, der Durchgang **(75)** nach aussen verschlossen, nämlich von der an der Toreinheit **(7)** dicht angedockten Behältnisaufnahme **(6),** die Aussenfläche der Abdeckung **(23),** der Zwischenraum **(70)** sowie alle diesem zugewandten Oberflächen steril und der Durchgang **(75)** in das Containment **(9)** offen; und nun
- der Zugang zum im Behältnis **(2)** liegenden Objekt **(3)** offen;
h) Entnehmen des Objekts **(3)** aus dem Behältnis **(2)** und Überführen des Objekts **(3)** mit den darin gespeicherten Containern **(30)** in die in Arbeitskammer **(90)** des Containments **(9)** zur weiteren Bearbeitung; hierbei sind:
- weiterhin der Zugangsflansch **(71)** der Toreinheit **(7)** mit dem Flansch **(61)** gegeneinander abgedichtet, der Durchgang **(75)** nach aussen verschlossen, nämlich von der an der Toreinheit **(7)** dicht angedockten Behältnisaufnahme **(6)** und der Durchgang **(75)** in das Containment **(9)** offen; und nun
- der Auffang **(60)** nur mehr mit dem leeren Behältnis **(2)** gefüllt; und
i) Rückführung der Anordnung in die Ausgangsphase, d.h. Bewegen der Tür **(5)** in die Schliessstellung, welche damit wieder gegen den Zugangsflansch **(71)** abgedichtet ist, Abschwenken der Behältnisaufnahme **(6)** vom Zugangsflansch **(71)** und Entfernen des leeren Behältnisses **(2)** aus dem Auffang **(60).**

9. Verfahren nach Anspruch 8, **dadurch gekennzeichnet, dass**
a) nach dem Heranfahren der Behältnisaufnahme **(6)** mit dem im Auffang **(60)** eingestellten Behältnis **(2)** an die Toreinheit **(7)** und vor dem Aktivschalten der Dekontaminationseinheit **(4)** bzw. Versetzen in ihren Wirkungszustand, das Objekt **(3)** einem Integritätstest unterzogen wird, um festzustellen, ob der Inhalt des Behältnisses **(2)** das Reinheitserfordernis erfüllt; wobei
b) falls der Integritätstest ein Negativergebnis zeigt, die Behältnisaufnahme **(6)** von der Toreinheit **(7)** abgeschwenkt wird, um das quasi schadhafte Behältnis **(2)** aus dem Auffang **(60)** zu entfernen.

10. Verfahren nach Anspruch 8, **dadurch gekennzeichnet, dass** das Aufschneiden der Abdeckung **(23)** des Behältnisses **(2),** die Entnahme des Objekts **(3)** aus dem Behältnis **(2)** und das Überführen des Objekts **(3)** mit den darin gespeicherten Containern **(30)** in die Arbeitskammer **(90)** des Containments **(9)** mittels des in der Arbeitskammer **(90)** positionierten Transfergeräts **(95)** erfolgt.

11. Verfahren nach Anspruch 10, **dadurch gekennzeichnet, dass** ein freigeschnittener Teil der Abdeckung **(23)** mittels des in der Arbeitskammer **(90)** positionierten Transfergeräts **(95)** in die Arbeitskammer **(90)** gebracht wird.

12. Verfahren nach zumindest einem der Ansprüche 8 bis 11, **dadurch gekennzeichnet, dass**
a) das einzelne Behältnis **(2),** welches mit der Abdeckung **(23)** verschlossen ist, mit seinem sterilen Inhalt, nämlich dem im Behältnis **(2)** gelagerten Objekt **(3)** und den darin aufgenommenen Containern **(30),** mit zumindest einer Umhüllung **(21,22)** versehenen ist und so im Anlieferungszustand eine Packung **(1)** bildet; und
b) die Bereitstellung des einzelnen Behältnisses **(2)** vor dem Einstellen in die Behältnisaufnahme **(6)** in einer zur Anordnung gehörenden Eingangsstation **(8)** als Freisetzen des Behältnisses **(2)** aus der jeweiligen Packung **(1)** unter kontrollierten reinraumtechnischen Bedingungen erfolgt.

## Claims

1. Assembly comprising a process chamber **(90)** in a containment **(9)** enclosed by a wall **(91)** and intended for the contamination-free introduction of a sterile object **(3)** into said process chamber **(90),** including:
a) a door unit **(7)** consisting of:
aa) an access flange **(71)** positioned in the wall **(91)** forming a passage **(75)** from the outside into the process chamber **(90);** and
ab) a door **(5)** that closes and seals the passage **(75)** and swings into open position in the process chamber **(90);**
b) a container holder **(6)** consisting of:
ba) a retainer **(60)** accessible through an opening **(65)** for retaining a container **(2)** inserted into the container holder **(6),** the container being closed by a lid **(23);** and
bb) a flange **(61),** operating in conjunction with the access flange **(71), characterized by** the fact that
c) the assembly contains a decontamination unit **(4)** in which
d) the decontamination unit **(4)** is designed to decontaminate the outer surface of the lid **(23)** of the container **(2)** facing the door **(5)** when the door **(5)** is closed, the container holder **(6)** docked against the access flange **(71)** and the container **(2)** is placed in the container holder **(6).**

2. Assembly as described in Claim 1 **characterized by** the fact that
a) the door **(5),** in closed position, closes and seals the access flange **(71)** on the process chamber **(90)** side, and the access flange **(71)** encompasses an interspace **(70)** inside the passage **(75);** and
b) the preferably semi-permeable lid **(23)** of the container **(2)** placed in the container holder **(6)** is sealed between the flange **(61)** and the access flange **(71)** when the container holder **(6)** is pivoted against the access flange **(71).**

3. Assembly as described in at least one of Claims 1 or 2 **characterized by** the fact that the decontamination unit **(4),** whose direction of action is into the passage **(75),** is installed directly on the door **(5)** or laterally on the door unit **(7)** consisting of the door **(5)** and the access flange **(71).**

4. Assembly as described in at least one of Claims 1 to 3 **characterized by** the fact that the decontamination unit **(4)** is a source of radiation (e.g. UVC, UV, e-beam or pulse light) or a fumigation device (e.g. for nebulization of an H₂O₂ solution).

5. Assembly as described in at least one of Claims 2 to 4 **characterized by** the fact that the decontamination unit **(4)** is further usable for decontaminating the outwardly facing surface of the door **(5),** the interspace **(70)** and the surface of the access flange **(71)** facing the interspace **(70).**

6. Assembly as described in at least one of Claims 1 to 5 **characterized by** the fact that a transfer device **(95)** provided with a tool head **(96)** is intended to be used in the process chamber **(90)** when the door **(5)** is open, the container holder **(6)** is docked on the access flange **(71)** and the container **(2)** is positioned in the container holder **(6)** in the following manner:
a) the decontaminated part of the lid **(23)** on the container **(2)** is detached and moved into the process chamber **(90);**
b) any insert **(28)** optionally present in the container **(2)** and located above the object **(3)** is moved into the process chamber **(90);** and
c) the object **(3)** contained in the container **(2)** located in the container holder **(6)** is moved into the process chamber **(90).**

7. Assembly as described in at least one of Claims 1 to 6 **characterized by** the fact that an input station **(8)** with an air stream flowing through a filter **(80)** is associated with the assembly and intended to release the containers **(2)** and to supply said containers **(2)** under controlled clean-room conditions prior to placing them in the container holder **(6).**

8. Method for contamination-free transfer of a sterile object **(3)** from a container **(2)** into a containment **(9)** using the assembly as described in at least one of the preceding Claims, said method comprising the following sequence of process steps:
a) Removal of the container **(2)** closed with a lid **(23)** and containing an object **(3)** from enclosures **(21, 22**) at an entry station **(8)** under controlled cleanroom conditions;
b) Provision of the respective individual container **(2)** closed with the lid **(23)** with sterile object **(3)** placed therein for contamination-free transfer into a process chamber **(90)** of a containment **(9);** whereby:
- the passage **(75)** to the process chamber **(90)** is open to the outside but closed to the containment **(9)** by the door **(5),** the decontamination unit **(4)** is not in operation, the retainer **(60)** of the container holder **(6)** is open and empty, the container holder **(6)** is disengaged from the door unit **(7);**
c) Positioning of the provided single container **(2)** into the retainer **(60);** whereby:
- the passage **(75)** continues to be open to the outside and closed to the containment **(9)** by the door **(5),** the decontamination unit **(4)** is not in operation and the container holder **(6)** is detached from the door unit **(7),** but now the holder **(60)** is loaded with a container **(2);**
d) Moving the container holder **(6)** to the door unit **(7);** whereby:
- the flange **(61)** abuts against the access flange **(71)** of the door unit **(7)** to form a seal;
- the passage **(75)** to the containment **(9)** continues to be closed by the door **(5),** the decontamination unit **(4)** is not in operation and the retainer **(60)** is loaded with a container **(2);** and
- the passage **(75)** is also closed to the outside, namely by the container holder **(6)** docked against the door unit **(7)** to form a seal;
e) Activating the decontamination unit **(4)** or switching it to the active state; whereby:
- the access flange **(71)** of the door unit **(7)** continues to be sealed to the flange **(61),** the passage **(75)** into the containment **(9)** is closed by the door **(5),** the retainer **(60)** is loaded with a container **(2),** and the passage **(75)** is also closed to the outside, namely by the container holder **(6)** docked to the door unit **(7)** to form a seal; and
- the outer surface of the lid **(23)** facing the door **(5)** as well as the interspace **(70)** and all surfaces facing it are now sterilized;
f) Deactivating or switching off the decontamination unit **(4)** and opening the door **(5);** whereby:
- the flange **(61)** and the access flange **(71)** of the door unit **(7)** continue to be sealed together, the retainer **(60)** is loaded with a container **(2),** the passage **(75)** is closed to the outside, namely by the container holder **(6)** which is tightly docked to the door unit **(7),** and the outer surface of the lid **(23),** the interspace **(70)** as well as all surfaces facing it are sterile; and
- the passage **(75)** to the containment **(9)** is now open;
g) Sliding open the lid **(23)** of the container **(2);** whereby:
- the flange **(61)** and the access flange **(71)** of the door unit **(7)** continue to be sealed together, the retainer **(60)** is loaded with a container **(2),** the passage **(75)** to the outside is closed, namely by the container holder **(6)** being tightly docked to the door unit **(7),** the outer surface of the lid **(23),** the interspace **(70)** as well as all surfaces enclosing it are sterile and the passage **(75)** into the containment **(9)** is open; and
- the object **(3)** located in the container **(2)** now becomes accessible;
h) Removing the object **(3)** from the container **(2)** and transferring said object **(3),** including the containers **(30)** therein, into the process chamber **(90)** of the containment **(9)** for further processing; whereby:
- the flange **(61)** and the access flange **(71)** of the door unit **(7)** continue to be sealed together, the passage **(75)** to the outside is closed off, namely by the container holder **(6)** being tightly docked to the door unit **(7),** and the passage **(75)** into the containment **(9)** is open; and
- the retainer **(60)** is now only loaded with the empty container **(2);** and
i) Returning the assembly to the initial phase, i.e. moving the door **(5)** to the closed position, which is thus sealed again against the access flange **(71),** pivoting the container holder **(6)** away from the access flange **(71)** and removing the empty container **(2)** from the retainer **(60).**

9. Method as described in Claim 8 **characterized by** the fact that
a) after the container holder **(6)** with the container **(2)** positioned in the retainer **(60)** is moved to the door unit **(7)** and before the decontamination unit **(4)** is activated or put into its operating state, the object (3) is subjected to an integrity test to determine whether the contents of the container **(2)** meet the purity requirement; whereby
b) if the integrity test yields a negative result, the container holder **(6)** is swung away from the door unit **(7)** in order to remove the presumably damaged container **(2)** from the retainer **(60).**

10. Method as described in Claim 8 **characterized by** the fact that the sliding open of the lid **(23)** of the container **(2),** the removal of the object **(3)** from the container **(2)** and the transfer of the object **(3),** including its containers **(30),** into the process chamber **(90)** of the containment **(9)** is carried out by means of the transfer device **(95)** located in the process chamber **(90).**

11. Method as described in Claim 10 **characterized by** the fact that an exposed portion of the lid **(23)** is brought into the process chamber **(90)** by means of the transfer device **(95)** located in the process chamber **(90).**

12. Method as described in at least one of Claims 8 to 11 **characterized by** the fact that
a) the individual container **(2),** which is closed with the lid **(23),** is provided with its sterile contents, namely the object **(3)** stored in the container **(2)** and the containers **(30)** located in said object **(3)** having at least one enclosure **(21, 22)** and thus forming a package **(1)** being delivered; and
b) the provision of the individual container **(2)** before it is placed in the container holder **(6)** takes place in an entry station **(8)** belonging to the assembly as a release of the container **(2)** from the respective package **(1)** under controlled cleanroom conditions.

## Revendications

1. Dispositif équipé d'une chambre de travail **(90)** d'un confinement **(9)** entourée d'une paroi **(91)** et destinée à l'infiltration sans contamination d'un objet stérile **(3)** dans la chambre de travail **(90),** comprenant :
a) un groupe portail **(7)** avec :
aa) une bride d'accès **(71)** agencée dans la paroi **(91),** formant un passage **(75)** depuis l'extérieur dans la chambre de travail **(90)** ; et
ab) une porte **(5)** fermant de manière étanche le passage **(75),** pouvant être déplacée dans la chambre de travail **(90)** en position ouverte ;
b) un logement de contenant **(6)** avec :
ba) un réceptacle **(60)** accessible par une ouverture **(65)** pour la fixation d'un contenant **(2)** placé dans un logement de contenant **(6),** fermé par un couvercle **(23)** ; et
bb) une bride **(61)** pour l'interaction avec la bride d'accès **(71)** ; **caractérisé en ce que**
c) le dispositif contient une unité de décontamination **(4),** dans laquelle
d) l'unité de décontamination **(4)** est réalisée de façon à ce que, quand la porte **(5)** est fermée, le logement de contenant **(6)** est fixé à la bride d'accès **(71)** et le contenant **(2)** est placé dans le logement de contenant **(6),** une surface extérieure du couvercle **(23)** du contenant **(2)** faisant face à la porte **(5)** est décontaminée.

2. Dispositif selon la revendication 1, **caractérisé en ce que**
a) la porte **(5)** est appliquée à la bride d'accès **(71)** étanchéifiée par les côtés de la chambre de travail **(90)** en position fermée, et **en ce que** la bride d'accès **(71)** délimite un espace intermédiaire **(70)** placé dans le passage **(75),** et **en ce que**
b) le couvercle **(23),** qui est de préférence semi-perméable, du contenant **(2)** placé dans le logement de contenant **(6)** est étanchéifié par le logement de contenant **(6)** rapproché par pivotement de la bride d'accès **(71)** entre la bride **(61)** et la bride d'accès **(71).**

3. Dispositif selon au moins l'une des revendications 1 ou 2, **caractérisé en ce que** l'unité de décontamination **(4),** avec sens d'actionnement vers l'intérieur du passage **(75),** est installée directement au niveau de la porte **(5)** ou latéralement du groupe portail **(7)** présentant la porte **(5)** et la bride d'accès **(71).**

4. Dispositif selon au moins l'une des revendications 1 à 3, **caractérisé en ce que** l'unité de décontamination **(4)** est une source de rayonnement, par exemple UVC, UV, faisceau d'électrons ou lumière puisée, ou un dispositif d'injection de gaz, par exemple pour la nébulisation d'une solution de H₂O₂.

5. Dispositif selon au moins l'une des revendications 2 à 4, **caractérisé en ce que** l'unité de décontamination **(4)** peut également être utilisée pour la décontamination de la surface de la porte **(5)** orientée vers l'extérieur, de l'espace intermédiaire **(70)** et de la surface de la bride d'accès **(71)** orientée vers l'espace intermédiaire **(70).**

6. Dispositif selon au moins l'une des revendications 1 à 5, **caractérisé en ce qu'**un appareil de transfert **(95)** équipé d'une tête d'outil **(96)** placé dans la chambre de travail **(90)** est destiné à, quand la porte **(5)** est ouverte, avec le logement de contenant **(6)** fixé à la bride d'accès **(71)** et le contenant **(2)** placé dans le logement de contenant **(6)** :
a) détacher la partie décontaminée du couvercle **(23)** du contenant **(2)** et à l'amener dans la chambre de travail **(90)** ;
b) amener dans la chambre de travail **(90)** un insert **(28)** disponible en option dans le contenant **(2)** et placé sur l'objet **(3)** ; et
c) amener dans la chambre de travail **(90)** l'objet **(3)** placé dans le contenant **(2)** se trouvant dans le logement de contenant **(6).**

7. Dispositif selon au moins l'une des revendications 1 à 6, **caractérisé en ce qu'**une station d'entrée **(8)** avec un flux d'air traversant un filtre **(80)** est associée au dispositif, et sert à libérer les contenants **(2)** et à positionner les contenants **(2)** dans des conditions de salle blanche contrôlées, avant leur placement dans le logement de contenant **(6).**

8. Procédé pour l'infiltration sans contamination d'un objet stérile **(3)** d'un contenant **(2)** dans un confinement **(9)** en utilisant le dispositif selon au moins l'une des revendications précédentes, ledit procédé comprenant la séquence de phases de processus suivantes :
a) Prélèvement du contenant **(2)** fermé par un couvercle **(23)** et contenant un objet **(3)** depuis des enveloppes **(21,22)** dans des conditions de salle propre contrôlées au niveau d'une station d'entrée **(8)** ;
b) Positionnement du contenant **(2)** individuel fermé par le couvercle **(23)** avec l'objet stérile **(3)** qu'il contient pour l'infiltration sans contamination dans une chambre de travail **(90)** d'un confinement **(9)** ; avec :
- le passage **(75)** vers la chambre de travail **(90)** étant ouvert vers l'extérieur, mais fermé par la porte **(5)** dans le confinement **(9),** l'unité de décontamination **(4)** étant désactivée, le réceptacle **(60)** du logement de contenant **(6)** étant ouvert et vide, le logement de contenant **(6)** étant détaché du groupe portail **(7)** ;
c) Introduction des contenants **(2)** individuels dans le réceptacle **(60),** avec :
- le passage **(75)** restant ouvert vers l'extérieur, mais fermé par la porte **(5)** dans le confinement **(9),** l'unité de décontamination **(4)** étant désactivée et le logement de contenant **(6)** étant détaché du groupe portail **(7),** mais le réceptacle **(60)** étant maintenant rempli par un contenant **(2)** ;
d) Rapprochement du logement de contenant **(6)** du groupe portail **(7)** ; avec :
- la bride d'accès **(71)** du groupe portail **(7)** étant étanche par rapport à la bride **(61)** ;
- le passage **(75)** dans le confinement **(9)** restant fermé par la porte **(5),** l'unité de décontamination **(4)** étant désactivée et le réceptacle **(60)** étant rempli par un contenant **(2)** ; et
- le passage **(75)** étant également fermé vers l'extérieur, à savoir par le logement de contenant **(6)** hermétiquement fixé au groupe portail **(7)** ;
e) Activation ou mise en état d'actionnement de l'unité de décontamination **(4)** ; avec :
- la bride d'accès **(71)** du groupe portail **(7)** restant étanche par rapport à la bride **(61),** le passage **(75)** dans le confinement **(9)** restant fermé par la porte **(5),** le réceptacle **(60)** étant rempli par un contenant **(2)** et le passage **(75)** étant également fermé vers l'extérieur, à savoir par le logement de contenant **(6)** fixé de manière hermétique au groupe portail **(7) ;** et
- la surface extérieure du couvercle **(23)** exposée vers la porte **(5)** ainsi que l'espace intermédiaire **(70)** et toutes les surfaces lui faisant face étant stérilisées ;
f) Arrêt ou mise hors état d'actionnement de l'unité de décontamination **(4)** et ouverture de la porte **(5),** avec :
- la bride d'accès **(71)** du groupe portail **(7)** restant étanche par rapport à la bride **(61),** le réceptacle **(60)** étant rempli par un contenant **(2),** le passage **(75)** étant fermé vers l'extérieur, à savoir par le logement de contenant **(6)** fixé de manière hermétique au groupe portail **(7),** et la surface extérieure du couvercle **(23),** de l'espace intermédiaire **(70)** et toutes les surfaces lui faisant face sont stériles ; et
- le passage **(75)** dans le confinement **(9)** étant ouvert ;
g) Découpe du couvercle **(23)** du contenant **(2)** ; avec :
- la bride d'accès **(71)** du groupe portail **(7)** restant étanche par rapport à la bride **(61),** le réceptacle **(60)** étant rempli par un contenant **(2),** le passage **(75)** étant fermé vers l'extérieur, à savoir par le logement de contenant **(6)** fixé de manière hermétique au groupe portail **(7),** la surface extérieure du couvercle **(23),** de l'espace intermédiaire **(70)** et toutes les surfaces lui faisant face étant stériles et le passage **(75)** dans le confinement **(9)** étant ouvert ; et
- l'accès à l'objet **(3)** se trouvant dans le contenant **(2)** étant ouvert ;
h) Prélèvement de l'objet **(3)** dans le contenant **(2)** et transfert de l'objet **(3)** avec les récipients **(30)** qu'il contient dans la chambre de travail **(90)** du confinement **(9)** pour leur traitement ultérieur ; avec :
- la bride d'accès **(71)** du groupe portail **(7)** reste étanche par rapport à la bride **(61),** le passage **(75)** étant fermé vers l'extérieur, à savoir par le logement de contenant **(6)** fixé de manière hermétique au groupe portail **(7)** et le passage **(75)** dans le confinement **(9)** étant ouvert ; et
- le réceptacle **(60)** ne contenant maintenant que le contenant **(2)** vide ; et
i) le retour du dispositif à la phase initiale, à savoir le déplacement de la porte **(5)** en position fermée, laquelle est ainsi de nouveau étanche par rapport à la bride d'accès **(71),** l'éloignement par pivotement du logement de contenant **(6)** de la bride d'accès **(71)** et l'enlèvement du contenant **(2)** vide du réceptacle **(60).**

9. Procédé selon la revendication 8, **caractérisé en ce que**
a) après le rapprochement du logement de contenant **(6)** avec le contenant **(2)** placé dans le réceptacle **(60)** du groupe portail **(7)** et avant l'activation de l'unité de décontamination **(4)** ou son déplacement en état d'actionnement, l'objet **(3)** est soumis à un test d'intégrité, afin de déterminer si le contenu du contenant **(2)** répond à l'exigence de pureté ; dans lequel
b) si le test d'intégrité donne un résultat négatif, le logement de contenant **(6)** est éloigné par pivotement du groupe portail **(7),** afin de sortir le contenant **(2)** susceptible d'être défectueux du réceptacle **(60).**

10. Procédé selon la revendication 8, **caractérisé en ce que** la découpe du couvercle **(23)** du contenant **(2),** le prélèvement de l'objet **(3)** dans le contenant **(2)** et le transfert de l'objet **(3)** avec les récipients **(30)** qu'il contient dans la chambre de travail **(90)** du confinement **(9)** a lieu au moyen du dispositif de transfert **(95)** placé au centre de la chambre de travail **(90).**

11. Procédé selon la revendication 10, **caractérisé en ce qu'**une partie librement découpée du couvercle **(23)** est amenée dans la chambre de travail **(90)** au moyen du dispositif de transfert **(95)** placé au centre de la chambre de travail **(90).**

12. Procédé selon au moins l'une des revendications 8 à 11, **caractérisé en ce que**
a) le contenant **(2)** individuel, fermé par le couvercle **(23),** avec son contenu stérile, à savoir l'objet **(3)** placé dans le contenant **(2)** et les récipients **(30)** qui y sont logés, est muni d'au moins une enveloppe **(21,22),** et forme ainsi un emballage **(1)** à l'état de livraison ; et
b) le positionnement des contenants **(2)** individuels avant leur placement dans le logement de contenant **(6)** a lieu dans une station d'entrée **(8)** faisant partie du dispositif et consiste en la libération du contenant **(2)** de l'emballage **(1)** correspondant dans des conditions de salle blanche contrôlées.
